# EUROPEAN PATENT APPLICATION

(11) **EP 1 836 958 A1**
(43) Date of publication of application: **26.09.2007**
(21) Application number: 07251178.5
(22) Date of filing: 20.03.2007
(51) Int. Cl.: A61B 5/06

(54) **A template for user in a surgical procedure**

(30) Priority: 23.03.2006 GB 0605807
(71) Applicant: Depuy International Limited, Beeston, Leeds LS11 8DT (GB); Biosense Webster, Diamond Bar CA 91765 (US); DePuy Orthopädie GmbH, 85551 Heimstetten (DE)
(72) Inventor: Baldewein, Jury c/p Depuy I-Orthopaedics, 85551 Heimstetten (DE); Slomczykowski, Mike c/o Depuy International Ltd, Leeds LS11 0EA (GB); Nitzan, Yaacov c/o Biosense Webster (Israel) Ltd., 39120 Tirat Hacarmel (IL); Revie, Ian c/o Depuy International Limited, Leeds, LS11 0EA (GB)
(74) Representative: Alton, Andrew

(57) **Abstract**

A template for use in a surgical procedure is described. The template comprises a template body, means for fixing the template body to a patient's tissue, a feature on the template body to indicate the appropriate location of the template body relative to an anatomical landmark, a feature on the template body to indicate the required location of a sensor which is to be implanted in the patient during the surgical procedure, and a feature on the template body for indicating the required location of a field generator which can be fixed to the template.

## Description

This invention relates to a template for use in a surgical procedure.

Sensors can be implanted in a patient for applications such as measuring temperature and analysing material composition. It can be particularly useful in many surgical procedures to use a sensor as a marker to provide location information. The sensor might then be tracked by a tracking system. A tracking sensor of this kind can be used in a catheter while it is navigated through a patient's vessels. It can be used in orthopaedic procedures in which the positions of instruments and implants relative to a patient's bone tissue are monitored. Accurate implantation of a sensor relative to the site of a surgical procedure can be important.

A sensor which is used to provide location information can make use of electromagnetic signals. US-5391199 and US-5443489 disclose details of systems which are applicable to this aspect of the present invention, in which the coordinates of a probe are determined using one or more field transducers, such as Hall effect devices, coils or other antennae carried on the probe. The sensor can include a coil, which can generate signals in response to externally-applied magnetic fields. The magnetic fields are generally by magnetic field transducers, such as radiator coils, fixed to an external reference frame in known locations. Systems which are concerned with tracking a sensor in 3-dimensional space are also disclosed in WO-96/05768, US-6690963 and US-A-2002/0065455. Subject matter that is disclosed in the specifications of these documents is incorporated in the specification for all purposes by these references.

US-A-2005/0245821 discloses magnetic tracking systems for generating locating information about objections which are used in a surgical procedure, including instruments and implants. The disclosed systems include one or more location pads which can be attached to the patient, and one or more position transducers which can be implanted in the patient's body. In some embodiments, the location pads transmit magnetic fields which are received by the transducers, in other embodiments, the transducers inside the body transmit magnetic fields which are received by the location pads. In each case, the received field amplitudes are used to determine the coordinates of the transducers in the body relative to one or more of the location pads.

Typically, each location pad is attached to the body surface close to the area in which the position transducer is located. As a result, accurate coordinates may be determined while transmitting relatively weak magnetic fields, and interference with the tracking system from close metal objects is reduced. There is no limitation on movement of the patient's body during the surgical procedure because the location pad moves together with the body.

Accurate location of a location pad relative to the site of a surgical procedure and to the site of an implanted sensor can be important.

The present invention provides a template for use in a surgical procedure, which comprises:
a. a template body,
b. means for fixing the template body to a patient's tissue,
c. a feature on the template body to indicate the appropriate location of the template body relative to an anatomical landmark,
d. a feature on the template body to indicate the required location of a sensor which is to be implanted in the patient during the surgical procedure,
e. a feature on the template body for indicating the required location of a field generator which can be fixed to the template.

The template facilitates accurate location of an implanted sensor relative to the site of a surgical procedure, and accurate location of an associated field generator relative to the site of the surgical procedure and to the site of an implanted sensor.

Furthermore, the template of the invention can facilitate accurate orientation of the field generator relative to the location of the implanted sensor.

It can be preferred for the template to include a plurality of removable strips of adhesive tape. These can be used to fix cables against movement during the procedure, for example by fixing the cables to the patient's body or to drapes or to other surfaces.

The template can include at least two sensor location features and at least two field generator fixation features. The template can then be used to facilitate location of implanted sensors and associated field generators on opposite sides of the site of a surgical procedure, especially in relation to a procedure to replace an orthopaedic joint, in which sensors and associated field generators are located in relation to the two bones on opposite sides of the joint.

Preferably, the template body has first and second limbs, with the template body location feature provided between the first and second limbs. For example, when the template is intended for use in a procedure for replacement of an orthopaedic joint, a first limb can be used to locate a sensor and an associated field generator with respect to one of the bones at the joint, and the second limb can be used to locate a sensor and an associated field generator with respect to other of the bones at the joint. It can be preferred for the template to include a hinge region which facilitates relative pivoting movement between its first and second limbs.

Preferably, the template includes at least one sensor location feature and at least one field generator fixation feature on each of the first and second limbs.

Preferably, the feature on the template body for indicating the required location of a sensor can be provided in the form of a hole extending through the body, or a mark which indicates where such a hole should be formed. The hole can receive a cutting tool such as a drill which be used to create a space in which the sensor can be implanted.

Preferably, the feature on the template body for indicating the required location of a field generator includes means for fixing the field generator to the template. For example, the template can include a quantity of one part of a hook and loop fastener material which can cooperate with a quantity of the other part of the hook and loop fastener material on the field generator to fasten the field generator to the template. Suitable hook and loop fastener material is available under the trade name Velcro.

Preferably, the template includes an indicator as to the correct orientation of the field generator relative to the location of the associated sensor. This can be provided in the form of a marking on the template against which the field generator can be aligned.

In another aspect, the invention provides an assembly for use in a surgical procedure, which comprises:
a. a template as discussed above,
b. a sensor which can be implanted in a patient, which is responsible to external electromagnetic fields for generating position data,
c. a field generator for generating an electromagnetic field for the sensor.

Preferably, the field generator has fastened to it a quantity of one part of a hook and loop fastener material, and in which the field generator fixation feature comprises a quantity of the other part of the hook and loop fastener material. Suitable hook and loop fastener material is available under the trade name Velcro.

The template can be used with an insertion instrument for a sensor. A suitable instrument is disclosed in International patent application no. PCT/GB2006/000614. Subject matter disclosed in the specification of that application is disclosed in this specification by this reference.

The template can be used as a component of a probe assembly which comprises a sensor which can be implanted in a patient responsive to external electromagnetic fields for generating position data, an insertion tool for the sensor, which can be used to manipulate the sensor and to deliver it to the desired implantation site, and a field generator for generating an electromagnetic field for the sensor. Preferably, the field generator and the insertion tool have mating formations by which they can be assembled together. Such a probe assembly is disclosed in the UK patent application which is filed with the present application, titled Probe Assembly and bearing agents' reference P211293. Subject matter disclosed in the specification of that application is disclosed in this specification by this reference.

The template should generally be formed from a material which is resistant to damage when exposed to body fluids. For some applications, it will be preferred that it is capable of being flexed, for example for use in relation to a joint replacement procedure when the joint should be flexed after the template has been fixed to it. Suitable materials might include coated papers, and polymers such as polyolefins, polyesters and the like.

Embodiments of the present invention will now be described by way of example with reference to the accompanying drawings, in which:
Figure 1 shows a sensor assembly comprising an implantable sensor and an associated field generator.
Figure 2 is a plan view of the template of the invention.

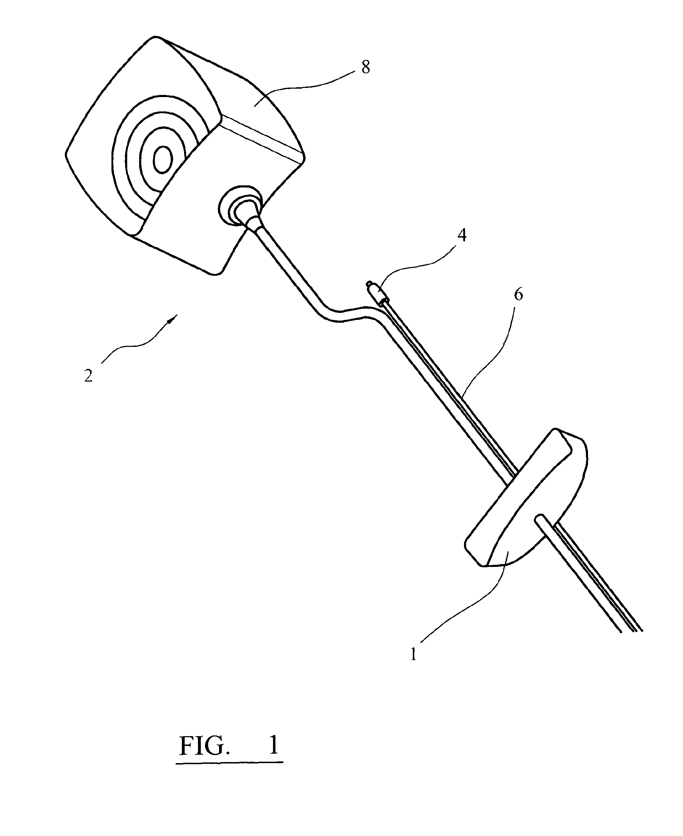

Referring to the drawings, Figure 1 shows a sensor assembly 2 which can be used in a surgical procedure. The assembly comprises an implantable sensor 4, which has a signal cable 6 extending from it. The sensor assembly also includes a field generator 8. The field generator can generate an electro-magnetic field for the sensor. The sensor 4 is able to generate position data in response to the externally applied electro-magnetic field from the field generator 8. Sensor assemblies of the kind shown in Figure 1, which include a transducer sensor and a location pad field generator are known from commercial products, for example as sold by DePuy International Limited and related companies. Information about such assemblies their uses is disclosed in US-A-2005/0245821 and International patent application no. PCT/GB2006/000614.

In use in an orthopaedic surgical procedure, the sensor 4 is implanted in a bore in a bone using an implantation tool, with the cable 6 extending from the sensor and through a channel in the overlying soft tissue. The field generator 8 is located on the surface of the patient tissue, close to the area in which the sensor is implanted. As a result, accurate coordinates may be determined while transmitting relatively weak magnetic fields, and interference with the tracking system from close metal objects is reduced. There is no limitation on movement of the patient's body during the surgical procedure because the field generator moves together with the body.

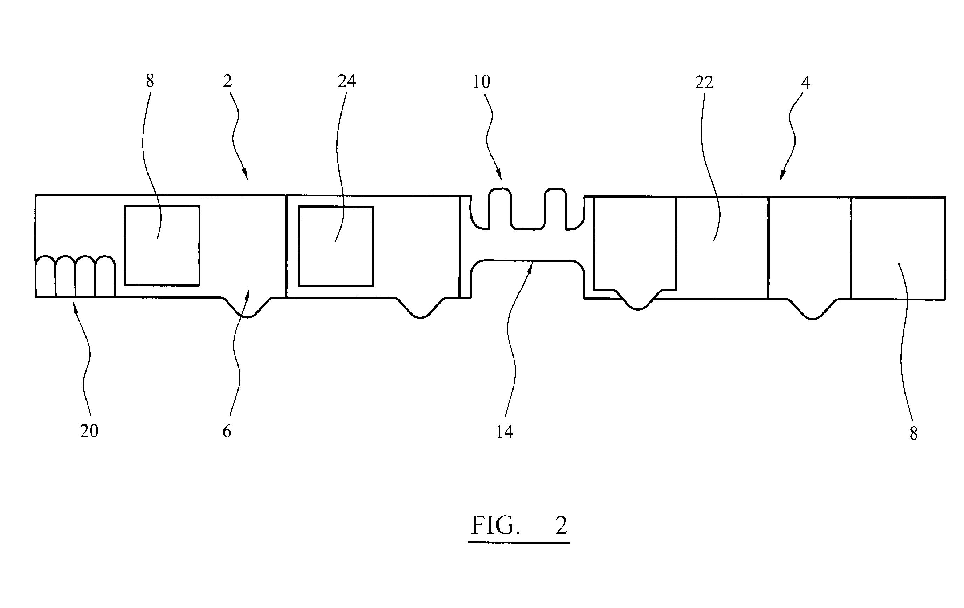

Figure 2 shows a template 2 has a first limb 4 and a second limb 6. Each of the limbs has a site 8 for mounting a field generator of a sensor assembly. Each of the sites 8 for mounting the sensor assembly has a quantity of one part of a hook and loop fastener material, for engaging with the other part of the hook and loop fastener material provided on the field generator. Suitable hook and loop fastener material is available under the trade name Velcro.

The template includes a hinge region 10 between the first and second limbs 4, 6. The template is flexible in the hinge region.

The template is provided on its lower surface (not shown) with a quantity of an adhesive material which can be used to fasten the template in place, generally on to the patient's skin. The adhesive material can be protected prior to use by means of a protective peelable sheet. The template can be provided in the hinge region with spaces between flexible fingers, which can facilitate separation of the peelable sheet from the template for removal thereof.

A position mark 14 is provided in the hinge region to indicate the appropriate location of the template relative to an anatomical landmark. When the template is intended for use in a procedure to replace an orthopaedic joint, the template will generally be positioned against the bones which are joined at the joint, with the position mark 14 located relative to the joint.

The template is provided with a plurality of strips 20 of adhesive tape. These strips can be removed from the template and used to fix cables against movement during the procedure, for example by fixing the cables to the patient's body or to drapes or to other surfaces.

The template has holes 22, 24 provided in it which mark the locations for a surgeon to make bores in the patient's tissue to receive the sensor for implantation. The holes are sized to receive a drill which can make a bore in the patient's bone tissue.

## Claims

1. A template for use in a surgical procedure, which comprises:
a. a template body,
b. means for fixing the template body to a patient's tissue,
c. a feature on the template body to indicate the appropriate location of the template body relative to an anatomical landmark,
d. a feature on the template body to indicate the required location of a sensor which is to be implanted in the patient during the surgical procedure,
e. a feature on the template body for indicating the required location of a field generator which can be fixed to the template.

2. A template as claimed in claim 1, which includes a plurality of removable strips of adhesive tape.

3. A template as claimed in claim 1, which includes at least two sensor location features and at least two field generator fixation features.

4. A template as claimed in claim 1, in which the template body has first and second limbs, with the template body location feature provided between the first and second limbs.

5. A template as claimed in claim 4, which includes a hinge region which facilitates relative pivoting movement between the first and second limbs.

6. A template as claimed in claim 4, which includes at least one sensor location feature and at least one field generator fixation feature on each of the first and second limbs.

7. A template as claimed in claim 1, in which the feature on the template body for indicating the required location of a field generator includes means for fixing the field generator to the template.

8. An assembly for use in a surgical procedure, which comprises:
a. a template as claimed in claim 1,
b. a sensor which can be implanted in a patient, which is responsible to external electromagnetic fields for generating position data,
c. a field generator for generating an electromagnetic field for the sensor.

9. An assembly as claimed in claim 8, in which the field generator has fastened to it a quantity of one part of a hook and loop fastener material, and in which the field generator fixation feature comprises a quantity of the other part of the hook and loop fastener material.
